# EUROPEAN PATENT SPECIFICATION

(11) **EP 1 500 706 B1**
(45) Date of publication and mention of the grant of the patent: **14.10.2009**
(21) Application number: 03746461.7
(22) Date of filing: 11.04.2003
(51) Int. Cl.: C12P 7/64, C11C 3/14, A23L 1/054, A23L 1/30, A23C 9/123, C12P 7/40

(54) **PROCESS FOR PRODUCING CONJUGATED FATTY ACID AND FOOD/DRINK OBTAINED BY THE PROCESS**
VERFAHREN ZUR HERSTELLUNG KONJUGIERTER FETTSÄURE UND MIT DIESEM VERFAHREN PRODUZIERTE LEBENSMITTEL/GETRÄNKE
PROCEDE DE PRODUCTION D'ACIDE GRAS CONJUGUE ET ALIMENT /BOISSON OBTENUS GRACE A CE PROCEDE

(30) Priority: 12.04.2002 JP 2002110773; 16.10.2002 JP 2002301286
(43) Date of publication of application: 26.01.2005
(60) Divisional application: 08154544.4
(73) Proprietor: KABUSHIKI KAISHA YAKULT HONSHA, Minato-ku, Tokyo 105-8660 (JP)
(72) Inventor: MIZUSAWA, Naomi Kabushiki Kaisha Yakult Honsha, Minato-ku, Tokyo 105-8660 (JP); SAKAI, Masashi Kabushiki Kaisha Yakult Honsha, Minato-ku, Tokyo 105-8660 (JP); KUDO, Satoshi Kabushiki Kaisha Yakult Honsha, Minato-ku, Tokyo 105-8660 (JP); SHIRASAWA, Yukio Kabushiki Kaisha Yakult Honsha, Minato-ku, Tokyo 105-8660 (JP)
(74) Representative: Texier, Christian
(86) International application number: PCT/JP2003/004633
(87) International publication number: WO 2003/087385

(56) References cited:
- EP-A- 1 174 416
- EP-A1- 1 264 893
- WO-A-99/29886
- WO-A-03/087344
- KR-A- 2001 089 858
- PATENT ABSTRACTS OF JAPAN vol. 2002, no. 06, 4 June 2002 (2002-06-04) & JP 2002 058425 A (YAKULT HONSHA CO LTD), 26 February 2002 (2002-02-26)
- SHIGENOBU KISHINO ET AL.: 'Conjugated linoleic acid production from linoleic acid by lactic acid bacteria' J. AM. OIL CHEM. SOC. vol. 79, no. 2, February 2002, pages 159 - 163, XP001086095
- JUN OGAWA ET AL.: 'Biseibutsu ni yoru kyoeki shibosan no seisan' YUKAGAKU TORONKAI YOSHISHU vol. 40, 2001, pages 53 - 54, XP002970298
- TUNG Y. LIN: 'Conjugated linoleic acid concentration as affected by lactic cultures and additives' FOOD CHEMISTRY vol. 69, no. 1, 2000, pages 27 - 31, XP002970299

## Description

### Technical Field

The present invention relates to a process according to claim 1.

### Background Art

Conjugated fatty acids are fatty acids each having double bonds at adjacent carbons with the interposition of a single bond. Among them, conjugated linoleic acids each containing 18 carbon atoms and having one conjugated diene in their molecule have been found to have various bioactivities.

The conjugated linoleic acids are industrially produced, for example, by alkali conjugation, in which an oil or fat containing linoleic acid, or free linoleic acid is conjugated in an organic solvent such as ethylene glycol.

In the alkali conjugation, an unsaturated fatty acid and excess alkali are generally heated to 150°C or higher in an organic solvent. The resulting conjugated fatty acid is a mixture of isomers having different bonding sites and/or configurations of double bonds. Linoleic acid as the raw material, for example, yields the cis-9, trans-11 isomer, trans-9, cis-11 isomer and trans-10, cis-12 isomer of conjugated linoleic acid, as well as some other positional isomers and/ or geometric isomers.

The bioactivities of the conjugated fatty acid are activities as a mixture of conjugated fatty acids. A technique for producing a specific conjugated fatty acid, if developed, has high academic and industrial importance. The alkali conjugation invites cyclization and other side reactions to decrease the yield of the conjugated fatty acid and to inhibit its purification.

Certain microorganisms and enzymes thereof have been reported to produce conjugated fatty acids. It has been reported, for example, that rumen bacteria produce conjugated fatty acids from a polyvalent unsaturated fatty acid (Shorland F. B., et al., Nature, vol. 175, p. 1129, 1955), Treponema (Yokoyama, et al., J. Bacteriology, vol. 107, pp. 519-527, 1971), Butyrivibrio fibrisolvens (Kepler C. R., et al., J. Biol. Chem., vol. 242, pp. 5686-92, 1967), Propionibacterium freudenreichii (Jiang J., Doctoral thesis, Swedish Uni. Uppsala, 1998), and ten and several percent of various pulmonary-pathogenic microorganisms (Jack C. I. A., et al., Clinica Chlimica Acts., vol 224, pp. 139-4, 1994) have a capability of isomerizing linoleic acid, and that these microorganisms produce conjugated linoleic acids mainly comprising the cis-9, trans-11 isomer.

Ruminant animals the cis-9, trans-11 isomer of conjugated linoleic acid in vivo, and dairy products and live-stock meat contain a large amount of the cis-9, trans-11 isomer of conjugated linoleic acid. Thus, the cis-9, trans-11 isomer of conjugated linoleic acid is believed to be a linoleic acid which human beings frequently take in general diet. A variety of investigations have been made on bioactivities of this isomer to find that the isomer has a defensive action against various cancers (Ha, Y. L., et al., Cancer Research vol. 50, p1097-1101, 1990, Ip C., et al., Cancer Research vol. 51, pp. 6118-6124).

As is described above, a product enriched for the cis-9, trans-11 isomer of conjugated linoleic acid having a promising anticarcinogenic activity is expected to be produced by using a microorganism. No microorganism that produces a sufficient amount of a conjugated linoleic acid with less by-products, however, has yet been found.

Lactic acid bacteria and bacteria belonging to the genus *Bifibacterium* are known as useful microorganisms for food. The bacteria belonging to the genus *Bifidobacterium* colonize in human colon, have various useful actions such as improvement in feculent and inhibition of enteral putrefaction and have been used in, for example, fermented milk.

The bacteria belonging to the genus *Bifidobacterium* are obligatory anaerobic and tend to die in the presence of oxygen or at a low pH. Thus, investigations using the bacteria belonging to the genus *Bifidobacterium* require an appropriate working environment and skilled operations. Thus, there has been little investigation on production of substances, including production of a conjugated linoleic acid, using the bacteria belonging to the genus *Bifidobacterium.*

Korean Patent Publication KR-A-2001-0089858 (published on October 12, 2001) proposes a fermented milk composition containing 0.001 - 5 wt% of a conjugated linoleic acid using a lactic acid bacterium, and a production process thereof.

As is described above, the alkali conjugation technique requires the reaction at elevated temperatures, often invites side reactions, is difficult to yield a specific conjugated fatty acid and requires a complicated purification step thereafter. The conversion reaction using a microorganism by-produces an all trans isomer of conjugated linoleic acid and produces conjugated linoleic acids in an insufficient total production amount.

The above-mentioned Korean Patent Publication discloses *Lactobacillus acidophilus, Lactobacillus casei, Bifidobacterium longum, Bifidobacterium adolescentis, Bifidobacterium breve* and *Bifidobacterium infantis* as the lactic acid bacteria producing a conjugated fatty acid in fermented milk compositions obtained by the use of such lactic acid bacteria. The publication also indicates that the production capability varies from strain to strain even in one species, and this is also indicated by investigations to achieve the present invention. No bacterial strain which produces 10 or more of the product based on 100 of the substrate linoleic acid has been obtained, although some bacterial strains produces 5 or more.

To solve these problems, the object of the present invention is to provide a process for producing a conjugated linoleic acid (CLA) enriched for the cis-9, trans-11 isomer, in which other conjugated linoleic acid isomers than the cis-9, trans-11 isomer of CLA occupy 10% or less of the total isomers of CLA.

### Disclosure of Invention

After intensive investigations to find a process for producing a conjugated linoleic acid with the use of a bacterium, the present inventors have found that certain bacteria belonging to the genus *Lactobacillus* and/or bacteria belonging to the genus *Bifidobacterium* are capable of conjugating, namely, are able to transfer the position of double bonds in the molecule of an unsaturated fatty acid and to isomerize into an isomer in which the double bonds are conjugated with each other.

According to the present invention, the above-mentioned objects can be achived by a process for producing a conjugated fatty acid comprising a step of conjugating an unsaturated fatty acid having at least two double bonds with the use of viable cells, dead cells or a cell extract of a bacterium having conjugation capability and belonging to the genus Lactobacillus and/or belonging to the genus *Bifidobacterium*, or an enzyme derived from the bacterium.

More specifically, the unsaturated fatty acid having at least two double bonds is conjugated by using viable cells, dead cells or a cell extract of at least one bacterium having conjugation capability selected from the group consisting of *Lactobacillus oris*, *Lactobacillus pontis, Lactobacillus panis*, *Bifidobacterium breve, Bifidobacterium bifidum, Bifidobacterium infantis* and *Bifidobacterium pseudocatenulatum*, or an enzyme derived from the bacterium.

The process according to the present invention conjugates an unsaturated fatty acid having at least two double bonds with the use of viable cells, dead cells or a cell extract of a bacterium belonging to the genus *Lactobacillus* and/ or a bacterium belonging to the genus *Bifidobacterium*, or an enzyme derived from the bacterium. Thus, the process can produce a conjugated fatty acid typified by the cis-9, trans-11 isomer of conjugated linoleic acid which is believed to have high bioactivities selectively in a high content and high yield.

*Lactobacillus oris* for use in the process for producing a conjugated fatty acid according to the present invention is a bacterium isolated from human saliva (Farrow J. A. E. et al., Int. J. Syst. Bacteriol. vol. 38, p. 116, 1988). The strain *Lactobacillus oris* NCDO 2160 (ATCC 49062), is used in the present invention.

*Lactobacillus pontis* is a bacterium isolated form rye bread leaven (Vogel, R. F., et al., Int. J. Syst. Bacteriol., vol. 44, pp. 223-229, 1994). *Lactobacillus pontis ATCC* 51518 and ATCC 51519, of which ATCC 51518 is preferred are used in the present invention.

*Lactobacillus panis* is a bacterium isolated from rye bread leaven (Sirohmar, W., Diekmann, H., Z. Lebensm. Unters. Forsch, vol. 194, pp. 536-540, 1992). *Lactobacillus panis JCM* 11053, is used in the present invention.

*Bifidobacterium breve* for use in the process for producing a conjugated fatty acid according to the present invention is a strain isolated from faeces and vaginalis of infants and suckling calves and are *Bifidobacterium breve* ATCC 15698, ATCC 15701 and YIT 10001, of which *Bifidobacterium breve* YIT 10001 is an excellent strain that can efficiently produces the cis-9, trans-11 isomer of conjugated linoleic acid in a high content. *Bifidobacterium* breveYIT 10001 was deposited as FERM P-18459 at international Patent Organism Depositary National Institute of Advanced Industrial Science and Technology on August 14, 2001 and was transferred to International Deposit FERM BP-8205 on October 11, 2002. *Bifidobacterium breve* YIT 10001 strain has a very high productivity of a conjugated linoleic acid and is preferred. This strain is capable of producing a conjugated linoleic acid in an amount of at least 0.5 mg/5-mL in a medium by preculturing in GAM broth (a product of Nissui Pharmaceutical Co., Ltd.) containing a complex between linoleic acid and BSA for at least 24 hours, inoculating 3.8 wt% of the cultured bacterium belonging to the genus *Bifidobacterium* to a milk medium containing a complex between linoleic acid and BSA and cultivating the bacterium with shaking for 144 hours. Such a strain having the excellent productivity can produce a conjugated linoleic acid to such an extent to expect bioactivities in a fermented food such as fermented milk when a food material such as milk material containing linoleic acid is fermented using the strain. Thus, the strain is typically suitable in, for example, food production with good operability

*Bifidobacterium pseudocatenulatum* is isolated from sewerage, the faeces of infants and faeces of suckling calves. *Bifidobacterium pseudocatenulatum* ATCC 27919 is used in the present invention.

The unsaturated fatty acid having at least two double bonds for use as a raw material in the present invention is linoleic acid. Linotoic acid is preferably used as the raw material to yield a conjugated linoleic acid. In this case, a specific isomer which has been reported to have bioactivities is specifically and efficiently produced with very trace amounts of other isomers (by-products).

The unsaturated fatty acid may be in the form of, for example, a salt or ester. Examples of the salt are salts of alkali metals, such as sodium salt and potassium salt; salts of alkaline earth metals, such as calcium salt and magnesium salt; and ammonium salts. Examples of the ester are methyl ester and ethyl ester. In addition, other lipids (phospholipids and glycolipids), monoglycerides, diglycerides and triglycerides each containing the unsaturated fatty acid can also be used.

Naturally occurring oils and fats can also be used as the raw material. Examples of those containing a large amount of linoleic acid in the molecule are naturally occurring oils and fats derived from vegetables, such as safflower oil, cotton seed oil, soybean oil, sunflower seed oil, corn oil, peanut oil, rice bran oil, linseed oil and cacao butter. Products of these decomposed by the action of a lipase can also be used as the raw material.

The bacterium is linoleic and cultured in a culture medium containing linoleic acid. A milk medium is preferably used as the culture medium.

The linoleic acid can be conjugated with the use of a bacterium listed above by any technique to produce CLA . Examples of such techniques are a technique of cultivating the bacterium in a growth medium containing linoleic acid to thereby produce a CLA directly; and a technique of cultivating the bacterium by any means, collecting and washing the resulting cells and adding the washed cells to a solution containing linoleic acid and allowing the cells to react to thereby produce CLA Not only viable cells of the bacterium, but also dead cells or a cell extract of the bacterium or an enzyme extracted from the cells can also be used for the reaction in a solution containing linoleic acid.

Any of media generally used for the growth of bacteria belonging to the genus *Lactobacillus* and/or bacteria belonging to the genus *Bifidobacterium*, or a milk medium containing milk can be used as the culture medium for cultivating the bacterium. The linoleic acid is preferably treated in a culture medium containing milk.

When a generally used growth medium such as MRS medium or GAM broth is used, the linoleic acid is not dispersed in the medium and the treatment is carried out inefficiently. Thus, the addition of a lipid-binding protein such as BSA (bovine serum albumin) or a surfactant, and/or homogenization under severe conditions is required. By using a milk medium, however, the material oil can be relatively easily homogenized without the addition of BSA, thus improving the operability and treatment efficiency and avoiding an increased cost due to the addition typically of BSA. When the present invention is applied to the production of a food containing a CLA, it is preferred to use a milk medium that can carry out the reaction efficiently even if the medium is free from BSA to thereby produce a fermented milk. This is because the lipid-binding protein such as BSA and the surfactant affects the flavor.

Such a milk medium can disperse the material oil excellently, probably because of the effects of protein components contained in the milk. The "milk" as used in the present description refers to lactoprotein-containing substances including raw milk, skim milk powder, whole milk powder or fresh cream of an animal milk such as cow's milk or goat's milk; as well as a vegetable milk such as soybean milk, almond milk or coconut milk.

To produce a conjugated linoleic acid, the bacterium is preferably precultured in a culture medium containing a complex between linoleic acid and at least one material selected from BSA, lipid-binding proteins and surfactants, although such a technique has the above-mentioned problems typically of cost. This configuration effectively increases the production of the conjugated linoleic acid in the main culture. The preculture is carried out for preferably 12 hours or more and more preferably 20 hours or more.

Washed cells, a cell powder, dead cells or a cell extract of the bacterium for use in the reaction can be prepared according to a conventional procedure. The washed cells, for be prepared by washing cultured cells with physiological saline or buffer. The cell powder can be prepared according to a drying technique such as freeze-drying or spray drying.

Examples of the techniques for preparing dead cells are a technique of allowing a cell-wall digesting enzyme, a technique of treating at a low osmosis, a technique of freeze-thawing, a technique of treating under high pressure, a technique of pulverization and a technique of heating. Among them, the technique of heating cells to allow autolysis can treat a large amount of cells at low cost and is preferred. The cell extract can be prepared by adding an appropriate solvent typically to the above-prepared washed cells, cell powder or dead cells and subjecting the mixture to centrifugal separation to yield the cell extract as a supernatant.

Examples of the conjugation process to yield CLA with the use of at least one bacterium listed above are the following processes (a) to (e):
(a) a process of cultivating the bacterium in a growth medium or milk medium (fermented milk) to conjugate linoleic acid in the form of fermentative production;
(b) a process of conjugating linoleic acid with the use of washed cells of the bacterium;
(c) a process of conjugating linoleic acid with the use of a cell powder of the bacterium;
(d) a process of conjugating linoleic acid with the use of dead cells of the bacterium; and
(e) a process of conjugating linoleic acid with the use of a cell extract of the bacterium.
The processes (a) to (e) will be illustrated in detail below.

### (a) Process of cultivating the bacterium in a growth medium or fermented milk to conjugate linoleic acid in the form of fermentative production:

The basic operation of conjugation in a growth medium or fermented milk may be carried out according to a conventional procedure. The following starter is preferably used.

Initially, linoleic acid is added to a lactic acid bacterium growth medium such as MRS medium (LACTOBACILLI MRS BROTH, a product of DIFCO) or a growth medium for anaerobe such as GAM medium (GAM broth, a product of Nissui Pharmaceutical Co., Ltd.) to induce the conversion ability efficiently in cell preparation. The concentration herein is in the range of 0 % to 0.2 % and preferably 0.05 % to 0.1 %.

This cultivation procedure is preferably stopped at pH 3.5 to 5.5, and typically preferably at pH 4.5 to 5.5. If the cultivation is continued to pH 3.5 or below, the capability of conjugating may decrease due to overgrowth of the bacterium. If the cultivation is continued at pH 5.5 or higher, no sufficient amount of cells to conjugate is obtained.

The resulting cultured mixture is preferably used as a starter. The starter is inoculated in an amount of preferably 0.5 % to 8 %, and typically preferably about 1 % to 4 % of a culture medium for the preparation of fermented milk. The amount of the raw material in the culture medium is preferably in the range of about 0.02 % to 0.8 % and typically preferably 0.1 % to 0.2 %. A culture temperature is in the range of about 20 °C to 40°C, and preferably 28 °C to 37°C.

For more efficiently yielding CLA, the cultivation is preferably stopped at pH 3.5 to 5.5 and more preferably at pH 4.5 to 5.5. The target CLA can also be prepared in a neutralization culture.

### (b) Process of conjugating linoleic acid with the use of washed cells of the bacterium:

Cells cultivated under the same conditions in the preparation of the starter in Process (a) are washed with physiological saline, collected and suspended in a buffer. Aqueous solutions that can maintain an appropriate pH are used as the buffer in which the cells are suspended and for the reaction of the washed cells. A 0.1 to 1.0 M phosphate buffer, for example, is preferably used. The pH of the buffer is 5.0 to 7.5 and preferably 6.0 to 7.0. The concentration of cells in the cell suspension is in the range of 0.025 % to 0.25 % and preferably 0.025 % to 0.1 % in terms of wet weight. The raw material has been mixed with bovine serum albumin (BSA) and is added to the reaction mixture in an amount of preferably in the range of about 0.1 % to 4.0 % and typically preferably 0.3 % to 1.0 %.

The amount of BSA is preferably about one fifth of that of the raw material. The temperature in the conversion reaction by the washed cells is 20 °C to 52 °C and preferably 32 °C to 37 °C. An appropriate time for conversion and production is 1 to 96 hours and preferably 24 to 72 hours. Cells cultivated while neutralizing the pH can also be used.

### (c) Process of conjugating linoleic acid with the use of a cell powder of the bacterium:

The cell powder of the bacterium can be prepared by cultivating the bacterium under the same conditions as in the preparation of the starter in Process (a), drying and powdering the resulting cells of the bacterium. The ceiis can be dried, for example, by freeze-drying or spray drying. The conversion reaction after the preparation of the cell powder may be carried out under the same conditions as in the reaction of washed cells in Process (b).

### (d) Process of conjugating linoleic acid with the use of dead cells of the bacterium:

The cells of the bacterium can be prepared by cultivating the bacterium under the same conditions as in the preparation of the starter in Process (a) and breaking the cell walls of the cells. The cell wall can be broken, for example, by any of a process of treating with an cell wall digestive enzyme, a process of suspending the cells in a solvent and treating the suspension at a low osmosis, a process of freezing and thawing, a process of treating under high pressure, a process of pulverization, and a process of heating. The conversion reaction after the preparation of the cell wall digestive product may be carried out under the same conditions as in the reaction for the washed cells in Process (b).

### (e) Process of conjugating linoleic acid with the use of a cell extract of the bacterium:

The cell extract of the bacterium can be prepared, for example, by extracting washed cells, cell powder or dead cells of the bacterium prepared by the procedure of Process (b), (c) or (d) with an appropriate solvent and removing the residue typically by centrifugal separation. The conversion reaction after the preparation of the cell extract may be carried out under the same conditions as in the reaction for the washed cells in Process (b).

The amounts of individual fatty acids in the fatty acid composition of the reaction mixture or cultured mixture obtained by the action of the bacterium, for example, by means of above-mentioned Process (a), (b), (c) or (d) can be determined by calculation based on the ratios of the areas of the individual fatty acids to that of an internal standard measured by gas chromatographic analysis. When the conjugated linoleic acids are the target compounds, gas chromatography may be carried out under the conditions shown in Table 1.

**Table 1**

| GC Analytical Condition | |
|---|---|
| GC system | GC-358 (a product of GL Sciences Inc.) |
| | GC14A (a product of Shimadzu Corporation) |
| Colum | DB-23 (ID 0.25 mm x 30 m) |
| Oven | 160 °C → 220 °C (at 2 °C/min) |
| Inj. Temp. | 250 °C |
| Det. Temp. | 250 °C |
| Carrier Gas | N₂ (50 mL/min) |
| Detector | FID |
| Sample Size | 1 µL in hexane |
| Sprit | 1/100 |

The CLA prepared according to the present invention can be administered in the form of, for example, pharmaceutical preparations, foods or cosmetics. In the case of a pharmaceutical preparation or a nutritional supplementary food which aims at bioactivities of a conjugated linoleic acid, the CLA can be orally administered in the dosage form of a solid preparation such as a capsule, granule, tablet or powder, or a liquid preparation such as syrup. The CLA can also be administered non-orally in the dosage form typically of an injection, skin external preparation or rectal preparation, instead of such an oral preparation.

Each preparation can be produced with the use of additives. Examples of such additives are excipients or fillers such as lactose, starch, crystalline cellulose, calcium lactate, magnesium aluminometasilicate and silicic anhydride; binders such as sucrose, hydroxypropyl cellulose and polyvinylpyrrolidone; disintegrators such as carboxymethylcellulose and calcium carboxymethylcellulose; lubricants such as magnesium stearate, talc, monoglyceride and sucrose fatty acid esters; as well as other ingredients that are acceptable typically as pharmaceutical preparations and/ or food.

When used in the form of a general food (in the form of an "apparent food") expecting similar bioactivities, such a food may be produced according to a conventional procedure by adding CLA obtained by the process of the present invention to a drink/food such as an oil, tablet-type sweets, fermented milk, candies, flavoring materials or dried food sprinkled over rice as intact or after appropriate purification. When used a fermented food, the fermented food can be produced by adding linoleic acid to a raw material for fermentation and fermenting (cultivating) the raw material by the action of a fermentative microorganism. It is preferred that milk containing linoleic acid is fermented to yield a fermented milk, since the resulting fermented milk can easily contain a large amount of the cis-9, trans-11 isomer of conjugated linoleic acid specifically, as described above.

A composition containing conjugated linoleic acid mainly comprising the cis-9, trans-11 isomer of conjugated linoleic acid can be obtained according to the present invention by using at least one selected from the group consisting of *Lactobacillus oris* ATCC 49062, *Lactobacillus pontis* ATCC 51518, *Lactobacillus pontis* ATCC 51519, *Lactobacillus panis* JCM 11053, *Bifidobacterium breve* YIT 10001, *Bifidobacterium breve* ATCC 15698, *Bifidobacterium breve* ATCC 15701, and *Bifidobacterium pseudocatenulatum* ATCC 27919. The "composition of conjugated linoleic acid enriched for the cis-9, trans-11 isomer" used in the present invention refers to a composition of conjugated linoleic acid in which other conjugated linoleic acid isomers than the cis-9, trans-11 isomer of conjugated linoleic acid occupies 10 % or less of the total isomers of conjugated linoleic acid. Such a composition has promising bioactivities such as anticancer activity even in a small amount and is excellent in taste, flavor and operability when added typically to a food. The ratio of the other isomers than the cis-9, trans-11 isomer of conjugated linoleic acid is more preferably 4% or less in the composition.

The "fermented milk" refers to and includes lactic drinks containing viable cells, such as fermented milk, milk products, lactic acid bacteria beverages; and lactic drinks containing sterilized fermented milk, as specified in the Ministerial Ordinance concerning Compositional Standards, etc. for Milk and Milk Products, the Ministry of Health, Labor and Welfare of Japan, as well as kefir. In the fermentation, other microorganisms including bacteria belonging to the genus *Lactobacillus,* such as *Lactobacillus casei, Lactobacillusacidophilus, Lactobacillus gasseri, Lactobacillus zeae, Lactobacillus johnsonii, Lactobacillus delbrueckii (ss. bulgaricus)* and *Lactobacillus delbrueckii (ss. detbrueckii);* bacteria belonging to the genus *Streptococcus*, such as *Streptococcus thermophilus;* bacteria belonging to the genus *Lactococcus,* such as *Lactococcus lactis (ss. lactis), Lactococcus lactis (ss*. *cremoris), Lactococcus plantarum* and *Lactococcus raffinolactis*; bacteria belonging to the genus *Leuconostoc*, such as *Leuconostoc mesenteroides* and *Leuconostoc lactis*; and bacteria belonging to the genus *Enterococcus*, such as *Enterococcus feacalis* and *Enterococcus faecium*.

Other microorganisms including bacteria belonging to the genus *Bifidobacterium*, such as *Bifidobacterium breve, Bifidobacterium bifidum, Bifidobacterium longum* and *Bifidobacterium animalis*, as well as yeasts can also be used. Each of these can be used alone or in combination.

These foods may further comprise other food materials such as carbohydrates, emulsifiers, thickeners, sweeteners, acidulants and fruit juice. Examples thereof are saccharides such as sucrose, isomerized sugar, glucose, fructose, paratinose, trehalose, lactose and xylose; sugar alcohols such as sorbitol, xylitol, erythritol, lactitol, paratinit, reduced starch syrup and reduced maltose syrup; emulsifiers such as sucrose fatty acid esters, glycerol fatty acid esters and lecithin; and thickeners (thickening stabilizers) such as carrageenan, Cyamoposis Gum, xanthan gum and Locust bean gum. In addition, vitamins such as vitamin A, and B vitamins; and minerals such as calcium, iron, manganese and zinc may also be incorporated.

The CLA produced by the process of the present invention can be appropriately incorporated into such pharmaceutical preparations and foods. When the bioactivities of CLA are required, the CLA may be incorporated in such an amount as to exhibit the bioactivities and avoid problems such as overdose, namely, such an amount as to take CLA in an amount in the range of about 10 mg to 1,000 mg per day.

### Brief Description of the Drawings

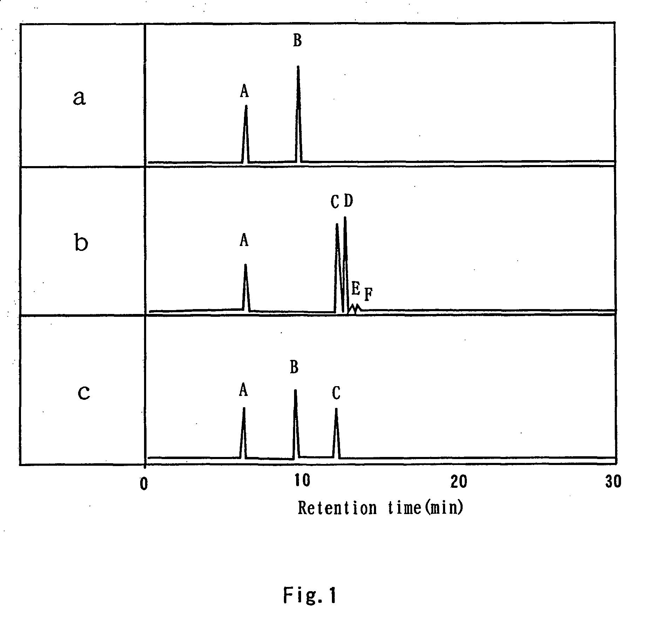

Fig. 1 shows gas chromatographic charts of conjugated linoleic acid isomers produced by *Lactobacillus oris,* in which graphs a, b and c are charts of a linoleic acid standard, a conjugated linoleic acid standard and a reaction mixture by the action of *Lactobacillus oris*, respectively. In the charts, Peak A shows the internal standard (17:0), Peak B linoleic acid, Peak C the cis-9, trans-11 isomer of conjugated linoleic acid, Peak D the trans-10, cis-12 isomer of conjugated linoleic acid, Peak E all-cis isomer of conjugated linoleic acid, and Peak F all-trans isomer of conjugated linoleic acid. The crowd of Peaks C, D, E and F shows the total isomers of conjugated linoleic acid.

### Best Mode for Carrying Out the Invention

The present invention will now be explained with reference to some examples below, which are not intended to limit the scope of the present invention.

### EXAMPLE 1: Screening of bacteria belonging to the genus Lactobacillus and producing CLA

In 1 mL of a 100 mM phosphate buffer (pH 6.5) were dissolved 50 mg of linoleic acid and 10 mg of BSA to yield a solution of a complex between linoleic acid and BSA.

Bacteria belonging to the genus *Lactobacillus* were inoculated to 15 mL of MRS medium (LACTOBACILLI MRS BROTH, a product of DIFCO) containing 0.07 % of linoleic acid and cultivated at 28 °C for 20 hours with shaking at 120 rpm. The cultured mixture had a pH of 4.7.

The cultured mixture was subjected to centrifugal separation to collect cells, and the cells were washed twice with physiological saline to yield washed cells. The washed cells were mixed with 100 µL of the solution of a complex between linoleic acid and BSA, and 0.9 mL of a 100 mM phosphate buffer (pH 6.5), and the mixture was reacted in an oxygen-impermeable plastic bag whose atmosphere was maintained anaerobic by using an agent for oxygen absorbing and carbon dioxide gas generating agent (Anaero Pack, a product of Mitsubishi Gas Chemical Company, Inc.) at 37 °C, 120 rpm for 24 hours.

The resulting reaction mixture was mixed with 1 mg of an internal standard (HEPTADECANOIC ACID), extracted by a Bligh-Dyer method, converted into a methyl ester (standing still in a 4 % solution of hydrochloric acid in methanol at room temperature for 30 minutes) and analyzed on fatty acids by gas chromatography.

Peaks of the conjugated linoleic acids were determined on the basis of the retention time of the standard (CLA 80, a product of RINORU OIL MILLS CO., LTD.), and their relative values were determined by calculation, taking linoleic acid added as the substrate as 100. The results in Table 2 show that *Lactobacillus oris* produces a conjugated linoleic acid. This strain, *Lactobacillus oris* NCDO 2160, has been registered as ATCC 49062 to the American Type Culture Collection (ATCC).

**Table 2**

| Strain | Conjugated linolic acid level, taking substrate linoleic acid as 100 |
|---|---|
| Control without bacterium | 0.1 |
| *Lactobacillus acidophilus* | 0.0 |
| *Lactobacillus brevis* | 0.1 |
| *Lactobacillus brevis* | 0.3 |
| *Lactobacillus casei* | 0.1 |
| *Lactobacillus casei* | 0.1 |
| *Lactobacillus gasseri* | 0.3 |
| *Lactobacillus johnsonii* | 0.0 |
| *Lactobacillus mali* | 0.0 |
| *Lactobacillus oris* NCD 2160. | 14.2 |
| *Lactobacillus reuderi* | 0.0 |
| *Lactobacillus reuderi* | 0.0 |
| *Lactobacillus rhamnosus* | 0.0 |
| *Lactobacillus rhamnosus* | 0.0 |
| *Lactobacillus sake* | 0.4 |

### EXAMPLE 2: Screening of bacteria belonging to the genus Bifidobacterium and producing CLA

In 1 mL of a 100 mM phosphate buffer (pH 6.5) were dissolved 50 mg of linoleic acid and 10 mg of BSA to yield a solution of a complex between linoleic acid and BSA. A total of 200 µL of the solution of a complex between linoleic acid and BSA was added to 15 mL of GAM broth (a product of Nissui Pharmaceutical Co., Ltd.), and fifteen strains of bacterium belonging to the genus *Bifidobacterium* were inoculated into the mixture, respectively, and cultivated at 35 °C for 48 hours with shaking at 120 rpm, to yield cultured media of bacteria belonging to the genus *Bifidobacterium.*

To 5mL of 10% skim milk medium (supplemented with 1 % of glucose and 0.1 % of soybean peptide) dispensed in 15-mL test tubes with a cap were added 100 µL (linoleic acid content: 5 mg) of the solution of a complex between linoleic acid and BSA and 200 µl of the above-prepared cultured media of the bacteria belonging to the genus *Bilido-bacterium,* respectively, and the cap was closed. The media were cultivated at 35 °C for 144 hours with shaking at 120 rpm to yield fermented milk products. The resulting fermented milk was mixed with 1 mg of an internal standard (HEPTADECANOIC ACID), extracted according to a Bligh-Dyer method, converted into a methyl ester (standing still in a 4 % solution of hydrochloric acid in methanol at room temperature for 30 minutes) and analyzed on fatty acids by gas chromatography. The results are shown in Table 3 below.

**Table 3**

| Fermented Milk Cultivated for 144 hours (Induction: linoleic acid) Unit (mg/5-mL) | | | | | | | | | | |
|---|---|---|---|---|---|---|---|---|---|---|
| Strain | YIT NO. | External registry No. | pH after cultivation | Linoleic acid | Conjugated linoleic acid | | | Hydroxy acid | | |
| | | | | | C9-t11 | T10-C12 | All trans | HOA 1 | HOA 2 | Others |
| B. adolescentis | 4011 | ATCC 15703 | 3.6 | 1.08 | 0.00 | 0.00 | 0.00 | 0.08 | 1.32 | 0.56 |
| B. adolescentis | 4087 | JCM 7042 | 3.6 | 1.04 | 0.00 | 0.01 | 0.00 | 0.10 | 1.57 | 0.33 |
| B. bifidum | 4007 | FERM BP-791 | 3.5 | 3.84 | 0.02 | 0.00 | 0.00 | 0.07 | 0.18 | 0.69 |
| B. bifidum | 4013 | | 5.2 | 1.41 | 0.00 | 0.00 | 0.00 | 0.00 | 0.00 | 0.85 |
| B. breve | 4064 | | 3.6 | 3.11 | 0.00 | 0.00 | 0.00 | 0.00 | 0.00 | 0.76 |
| B. breve | 4065 | | 3.6 | 1.93 | 0.00 | 0.00 | 0.00 | 0.00 | 0.00 | 0.46 |
| B. breve | 10001 | FERM BP-8205 | 3.5 | 0.61 | 0.57 | 0.00 | 0.02 | 0.00 | 0.00 | 0.31 |
| B. catenulatum | 4016 | ATCC 27539 | 3.3 | 2.99 | 0.00 | 0.00 | 0.00 | 0.06 | 0.49 | 0.66 |
| B. catenulatum | 4118 | JCM 7130 | 4.4 | 3.99 | 0.00 | 0.00 | 0.00 | 0.00 | 0.00 | 0.73 |
| B. infantis | 4018 | ATCC 15697 | 6.0 | 4.28 | 0.00 | 0.00 | 0.00 | 0.00 | 0.00 | 0.89 |
| B. infantis | 4019 | ATCC 15702 | 4.7 | 0.87 | 0.05 | 0.00 | 0.00 | 0.00 | 0.00 | 0.52 |
| B. lactis | 4121 | DSM 10140 | 4.3 | 3.81 | 0.00 | 0.00 | 0.00 | 0.05 | 0.00 | 0.61 |
| B. longum | 4021 | ATCC 15707 | 3.8 | 2.81 | 0.00 | 0.00 | 0.00 | 0.06 | 0.07 | 0.70 |
| B. longum | 4037 | ATCC 15708 | 3.6 | 0.99 | 0.00 | 0.00 | 0.00 | 0.00 | 0.56 | 0.27 |
| B. pseudocatennulatum | 4072 | ATCC 27919 | 3.7 | 4.02 | 0.02 | 0.00 | 0.00 | 0.00 | 0.00 | 0.99 |

Peaks of conjugated linoleic acids were determined and analyzed on the basis of the pretension time of a standard (CLA 80, a product of RINORU OIL MILLS CO., LTD.). As a result, *Bifidobacterium breve* YIT 10001 (FERM BP-8205), *Bifidobacterium infantis* ATCC 15702, *Bifidobacterium bifidum* FERM BP-791 and *Bifidobacterium pseudocatenulatum* ATCC 27919 were found to produce a conjugated linoleic acid.

Among them, *Bifidobacterium breve* YIT 10001 (FERM BP-8205) converts 11.4 % (= 0.57 mg/5mg x 100) of the added linoleic acid (5 mg), and most of (96 % or more (0.57/0/59 x 100) of the produced conjugated linoleic acids is the cis-9, trans-11 isomer of conjugated linoleic acid. However, *Bifidobacterium breve* YIT 4046 and *Bifidobacterium breve* YIT 4065 have no activity for producing a conjugated linoleic acid, although they belong to the species *Bifidobacterium breve,* showing that the production activity for a conjugated linoleic acid is present in a specific strain of *Bifidobacterium breve.*

### EXAMPLE 3: Identification of isomers in CLA produced by bacterium belonging to the genus Lactobacillus

The isomer of conjugated linoleic acid produced by *Lactobacillus oris* in Example 1 was analyzed. Fig. 1 is a gas chromatographic chart of the isomer of conjugated linoleic acid produced by *Lactobacillus oris,* showing that all the conjugated linoleic acid produced by *Lactobacillus oris* is the cis-9, trans-11 isomer of conjugated linoleic acid. The result shows that, among a multitude of lactic acid bacteria, *Lactobacillus oris* selectively and efficiently produces the cis-9, trans-11 isomer of conjugated linoleic acid in a high content.

### EXAMPLE 4: Production of CLA in fermented milk by bacterium belonging to the genus Lactobacillus

In 1 mL of a 100 mM phosphate buffer (pH 6.5) were dissolved 50 mg of linoleic acid and 10 mg of BSA to yield a solution of a complex between linoleic acid and BSA. A total of 200 µL of the solution of a complex between linoleic acid and BSA was added to 15 mL of MRS medium (LACTOBACILLI MRS BROTH, a product of DIFCO), and *Lactobacillus oris* was inoculated thereinto and cultivated at 28 °C for 20 hours with shaking at 120 rpm to yield a cultured mixture of pH 4.7.

To 5 mL of a 10 % skim milk medium (supplemented with 1 % of glucose and 0.1 % of soybean peptide) dispensed in 15-mL test tubes with a cap were added 100 µL of the solution of a complex between linoleic acid and BSA and 200 µL of the above-prepared cultured mixture of the individual strain, and the cap was closed. The medium was cultivated at 28°C for 48 hours with shaking at 120 rpm to yield a fermented milk. The resulting fermented milk was subjected to analysis on fatty acids by the same procedure of Example 1.

The result shows that 2.0 % of the added linoleic acid was converted into a conjugated linoleic acid, and that all the produced conjugated linoleic acid is the cis-9, trans-11 isomer of conjugated linoleic acid, as in Example 3. These results show that *Lactobacillus oris* selectively and efficiently produces the cis-9, trans-11 isomer of conjugated linoleic acid also in a fermented milk.

### EXAMPLE 5: Production of CLA in the reaction of washed cells of bacteria belonging to the genus Bifidobacterium

In 1 mL of a 100 mM phosphate buffer (pH 6.5) were dissolved 50 mg of linoleic acid and 10 mg of BSA to yield a solution of a complex between linoleic acid and BSA.

The solution of a complex between linoleic acid and BSA was added to 15 mL of GAM broth (a product of Nissui Pharmaceutical Co., Ltd.) in a proportion of 0.07 %, and *Bifidobacterium breve* YIT 10001 (FERM BP-8205) was inoculated to the mixture and cultivated at 35 °C for 48 hours with shaking at 120 rpm. The cultured mixture was separated by centrifugation to collect cells, and the cells were washed twice with physiological saline to yield washed cells.

The washed cells were mixed with 100 µL of the solution of a complex between linoleic acid and BSA and 0.9 mL of a 100 mM phosphate buffer (pH 6.5) in a test tube. After replacing the gas phase with nitrogen gas, the test tube was sealed to maintain the inside anaerobic, and a reaction was carried out at 37 °C, 120 rpm for 72 hours.

The resulting reaction mixture was mixed with 1 mg of an internal standard (HEPTADECANOIC ACID), extracted according to a Bligh-Dyer method, converted into a methyl ester (standing still in a 4% solution of hydrochloric acid in methanol at room temperature for 30 minutes) and analyzed on fatty acids by gas chromatography.

The result shows that *Bifidobacterium breve* YIT 10001 (FERM BP-8205) converts 0.9 % of added linoleic acid in the substrate into the cis-9, trans-11 isomer of conjugated linoleic acid.

### EXAMPLE 6: Production of CLA by dead cells of Lactobacillus oris

In 1 mL of a 100 mM phosphate buffer (pH 6.5) were dissolved 50 mg of linoleic acid and 10 mg of BSA to yield a solution of a complex between linoleic acid and BSA. A total of 200 µL of the solution of a complex between linoleic acid and BSA was added to 15 mL of MRS medium (LACTOBACILLI MRS BROTH, a product of DIFCO), and *Lactobacillus oris* was inoculated thereinto and cultivated at 28 °C for 20 hours with shaking at 120 rpm to yield a cultured mixture of pH 4.7.

To 15 mL of ILS medium supplemented with 0.3 % of glucose was added 0.5 mL of the above-prepared cultured mixture, and cultivated at 37 °C for 18 hours (to pH 5.6) with shaking at 120 rpm. Cells were collected by centrifugal separation and washed twice with a 0.2 M glycine buffer (pH 10.6). The washed cells were dissolved in 2 mL of a solution of 6.7 % sucrose - 50 mM tris (hydroxymethyl) aminomethane - 1 mM EDTA. The solution was mixed with 0.8 mL of a lysozyme solution (10 mg/mL in 25 mM tris (hydroxymethyl) aminomethane, pH 8.0, a product of SEIKAGAKU CORPORATION) and 0.15 mL of an aqueous solution of N-Acetylmuramidase (1 mg/mL, a product of SEIKAGAKU CORPORATION) and was reacted at 37 °C, 120 rpm for 30 minutes. The reaction mixture was transferred to Centriprep 10 (a product of Amicon Inc.) and concentrated to yield about 0.6 mL of a suspension of dead cells of *Lactobacillus oris*.

The suspension of dead cells was mixed with 100 µL of the solution of a complex between linoleic acid and BSA and 0.9 mL of a 100 mM phosphate buffer (pH 6.5), and the mixture was reacted in an oxygen-impermeable plastic bag whose atmosphere was maintained anaerobic by using an agent for oxygen absorbing and carbon dioxide gas generating (Anaero Pack, a product of Mitsubishi Gas Chemical Company, Inc.) at 37°C, 120 rpm for 24 hours.

The resulting reaction mixture was subjected to fatty acid analysis by the procedure of Example 1 to find that 1.6 % of the added linoleic acid was converted into a conjugated linoleic acid. All the produced conjugated linoleic acid is the cis-9, trans-11 isomer of conjugated linoleic acid, as in the washed cells and the fermented milk. These results show that *Lactobacillus oris* selectively and efficiently produces the cis-9, trans-11 isomer of conjugated linoleic acid even as dead cells whose cell walls have been broken.

### EXAMPLE 7: Production of CLA by Lactobacillus pontis

In 1 mL of a 100 mM phosphate buffer (pH 6.5) were dissolved 50 mg of linoleic acid and 10 mg of BSA to yield a solution of a complex between linoleic acid and BSA. A total of 200 µL of the solution of a complex between linoleic acid and BSA was added to 15 mL of MRS medium (LACTOBACILLI MRS BROTH, a product of DIFCO), and *Lactobacillus pontis* (ATCC 51518) was inoculated thereinto and cultivated at 28 °C for 48 hours with shaking at 120 rpm to yield a cultured mixture having a pH of 4.9.

To 5 mL of a 10 % skim milk medium dispensed and sterilized in a 15-mL test tube with a cap were added 100 µL of the solution of a linoleic acid-BSA complex and 400 µL of the above-prepared cultured mixture of the strain, and the cap was closed. The medium was cultivated at 28 °C for 48 hours with shaking at 120 rpm to yield a fermented milk having a pH of 5.5. The resulting fermented milk was subjected to analysis on fatty acids by the same procedure of Example 1.

The result shows that 0.5 % of the added linoleic acid was converted into a conjugated linoleic acid, and that all the produced conjugated linoleic acid was the cis-9, trans-11 isomer of conjugated linoleic acid. These results show that *Lactobacillus pontis* selectively and efficiently produces the cis-9, trans-11 isomer of conjugated linoleic acid in a high content.

### EXAMPLE 8: Preparation of a fermented food containing a conjugated linoleic acid with the use of Bifidobacterium breve YIT 10001 (FERM BP-8205)

A medium containing 10 % of skim milk powder, 1 % of glucose and 0.1 % of soybean peptide was supplemented with 0.1 % or 1.0 % of linoleic acid and homogenized at 150 kg/cm². The homogenized medium was sterilized in an autoclave at 115 °C for 10 minutes to yield a medium for the preparation of a fermented food.

*Bifidobacterium breve* YIT 10001 (FERM BP-8205) was inoculated into 15 mL of GAM broth (a product of Nissui Pharmaceutical Co., Ltd.) and cultivated at 35°C for 48 hours with shaking at 120 rpm to yield a cultured mixture of the bacterium belonging to the genus *Bifidobscterium.* A total of 6 mL of the cultured mixture was inoculated into 150 mL of the above-prepared medium for the preparation of a fermented food, the gas phase was replaced with nitrogen gas, and the medium was anaerobically cultivated while standing at 35 °C, 120 rpm for 72 hours to yield a fermented food.

The resulting reaction mixture was analyzed on fatty acids by the procedure of Example 2 to find that part of the added linoleic acid was converted into a conjugated linoleic acid, and that all the produced conjugated linoleic acid was the cis-9, trans-11 isomer of conjugated linoleic acid.

The prepared fermented food was subjected to an organoleptic test to find to be equivalent to a food containing no linoleic acid.

## Claims

1. Process for producing conjugated linoleic acid, comprising the step of conjugating linoleic acid, with the use of viable cells, dead cells or a cell extract of at least one bacterium having conjugation capability selected from the group consisting of Lactobacillus oris ATCC 49062, Lactobacillus pontis ATCC 51518, Lactobacillus pontis ATCC 51519, *Lactobacillus panis* JCM 11053, *Bifidobacterium breve* YIT 10001 (FERM BP-8205), *Bifidobacterium breve* ATCC 15698, *Bifidobacterium breve* ATCC 15701, and *Bifidobacterium pseudocatenulatum* ATCC 27919.

2. Process according to claim 1, comprising the steps of inoculating the bacterium having conjugation capability into a culture medium which contains linoleic acid and cultivating the bacterium in said culture medium.

3. The process according to claim 2 wherein a milk medium is used as the culture medium to yield a fermented milk containing a conjugated linoleic acid.

4. The process according to claim 3, wherein a milk medium being free of lipid-binding proteins and surfactants is used as the culture medium to yield a fermented milk containing conjugated linoleic acid.

5. Process according to claim 1 **characterized in that** the bacterium is capable of producing a conjugated linoleic acid in an amount exceeding 10, based on 100 of the substrate linoleic acid added to a culture medium, through the following steps:
(a) carrying out a preculture in a growth medium for at least 12 hours, the growth medium comprising a complex between linoleic acid and at least one material selected from BSA, lipid-binding proteins and surfactants; and
(b) inoculating a bacterium having conjugation capability obtained in the step (a) to a milk medium containing linoleic acid and cultivating the bacterium with shaking for a predetermined time and the bacterium is *Lactobacillus oris* ATCC 49062 or *Bifidobacterium breve* YIT 10001 (FERM BP-8205).

## Patentansprüche

1. Verfahren zur Herstellung konjugierter Linolsäure, umfassend den Schritt der Konjugation von Linolsäure mittels lebensfähiger Zellen, toter Zellen oder eines Zellextrakts wenigstens eines Bakteriums mit Konjugationsvermögen, das aus der Gruppe bestehend aus *Lactobacillus oris* ATCC 49062, *Lactobacillus pontis* ATCC 51518, *Lactobacillus pontis* ATCC 51519, *Lactobacillus panis* JCM 11053, *Bifidobacterium breve* YIT 10001 (FERM BP-8205), *Bifidobacterium breve* ATCC 15698, *Bifidobacterium breve* ATCC 15701 und *Bifidobacterium pseudocatenulatum* ATCC 27919 ausgewählt ist.

2. Verfahren nach Anspruch 1, umfassend die Schritte des Einimpfens des Bakteriums mit Konjugationsvermögen in ein Kulturmedium, das Linolsäure enthält, und der Kultivierung des Bakteriums in diesem Kulturmedium.

3. Verfahren nach Anspruch 2, wobei ein Milchmedium als Kulturmedium verwendet wird, um eine fermentierte Milch zu ergeben, die eine konjugierte Linolsäure enthält.

4. Verfahren nach Anspruch 3, wobei ein Milchmedium, das frei ist von lipidbindenden Proteinen und grenzflächenaktiven Stoffen, als Kulturmedium verwendet wird, um eine fermentierte Milch zu ergeben, die konjugierte Linolsäure enthält.

5. Verfahren nach Anspruch 1, **dadurch gekennzeichnet, dass** das Bakterium imstande ist, eine konjugierte Linolsäure in einer Menge herzustellen, die 10 bezogen auf 100 des einem Kulturmedium zugesetzten Substrats Linolsäure überschreitet, und zwar durch die folgenden Schritte:
(a) Durchführen einer Vorkultur in einem Nährmedium für wenigstens 12 Stunden, wobei das Nährmedium einen Komplex zwischen Linolsäure und wenigstens einer Substanz, die unter BSA, lipidbindenden Proteinen und grenzflächenaktiven Mitteln ausgewählt ist, umfasst; und
(b) Einimpfen eines in Schritt (a) erhaltenen Bakteriums mit Konjugationsvermögen in ein Milchmedium, das Linolsäure enthält, und Kultivieren des Bakteriums unter Schütteln für eine vorbestimmte Zeit,
und das Bakterium *Lactobacillus oris* ATCC 49062 oder *Bifidobacterium breve* YIT 10001 (FERM BP-8205) ist.

## Revendications

1. Procédé de production d'un acide linoléique conjugué, comprenant l'étape consistant à conjuguer l'acide linoléique, avec l'utilisation de cellules viables, de cellules mortes ou d'un extrait cellulaire d'au moins une bactérie capable de se conjuguer choisie dans le groupe constitué par Lactobacillus oris ATCC 49062, Lactobacillus pontis ATCC 51518, Lactobacillus pontis ATCC 51519, Lactobacillus panis JCM 11053, Bifidobacterium breve YIT 10001 (FERM BP-8205), Bifidobacterium breve ATCC 15698, Bifidobacterium breve ATCC 15701 et Bifidobacterium pseudocatenulatum ATCC 27919.

2. Procédé selon la revendication 1, comprenant les étapes consistant à inoculer la bactérie capable de se conjuguer dans un milieu de culture contenant de l'acide linoléique et à cultiver la bactérie dans ledit milieu de culture.

3. Procédé selon la revendication 2, dans lequel un milieu à base de lait est utilisé comme milieu de culture pour donner un lait fermenté contenant un acide linoléique conjugué.

4. Procédé selon la revendication 3, dans lequel un milieu à base de lait ne contenant pas de protéines se liant aux lipides et de tensioactifs est utilisé comme milieu de culture pour donner un lait fermenté contenant un acide linoléique conjugué.

5. Procédé selon la revendication 1, **caractérisé en ce que** la bactérie est capable de produire un acide linoléique conjugué en une quantité dépassant 10 sur la base de 100 du substrat acide linoléique ajouté à un milieu de culture, par l'intermédiaire des étapes suivantes .
(a) réaliser une préculture dans un milieu de croissance pendant au moins 12 heures, le milieu de croissance comprenant un complexe entre l'acide linoléique et au moins une substance choisie parmi la BSA, des protéines se liant aux lipides et des tensioactifs ; et
(b) inoculer une bactérie capable de se conjuguer obtenue dans l'étape (a) dans un milieu à base de lait contenant de l'acide linoléique et cultiver la bactérie avec secouage pendant une durée prédéterminée, et la bactérie est Lactobacillus oris ATCC 49062 ou Bifidobacterium breve YIT 10001 (FERM BP-8205).
